# EUROPÄISCHE PATENTSCHRIFT

(11) **EP 3 964 822 B1**
(45) Veröffentlichungstag und Bekanntmachung des Hinweises auf die Patenterteilung: **01.11.2023**
(21) Anmeldenummer: 20194405.5
(22) Anmeldetag: 03.09.2020
(51) Int. Cl.: G01N 21/66, G01N 21/67, G01N 21/68, A61B 5/00, A61B 18/12, A61B 17/00, A61B 18/00

(54) **EINRICHTUNG UND VERFAHREN ZUR GEWEBEANALYSE**
DEVICE AND METHOD FOR TISSUE ANALYSIS
DISPOSITIF ET PROCÉDÉ D'ANALYSE TISSULAIRE

(43) Veröffentlichungstag der Anmeldung: 09.03.2022
(73) Patentinhaber: Erbe Elektromedizin GmbH, 72072 Tübingen (DE)
(72) Erfinder: JÄGER, Jan, 72074 Tübingen (DE); DANIEL, Yannick, 72336 Balingen (DE)
(74) Vertreter: Rüger Abel Patentanwälte PartGmbB

(56) Entgegenhaltungen:
- EP-A1- 2 815 695
- EP-A1- 3 685 781

## Beschreibung

Die Erfindung betrifft eine Einrichtung sowie ein Verfahren zur Gewebeanalyse, insbesondere zur Integration in eine chirurgische Einrichtung.

Es ist bekannt, mittels elektrischer Funken oder elektrisch erzeugten Plasmas auf biologisches Gewebe einzuwirken und das dabei entstehende Licht zu analysieren, um Rückschlüsse auf das behandelte Gewebe zu ziehen. Die WO 2011/055369 A2 offenbart dazu ein Katheter zur Plasmaablation, wobei der Katheter eine Lichtaufnahmeeinrichtung in Gestalt einer optischen Faser aufweist, die in der Nähe einer Ablationselektrode angeordnet ist. Das von der optischen Faser aufgenommene Licht wird einer Analyseeinrichtung, beispielsweise einem Spektrometer, zugeführt und einer Spektralanalyse unterzogen. Um insbesondere unterscheiden zu können, ob der elektrische Funke auf Ablagerungen, sogenannte Plaques, oder auf lebende Zellen, zum Beispiel Blut, einwirkt, wird insbesondere die Phosphorlinie des Spektrums beobachtet (254 nm).

Die EP 2 815 695 A1 offenbart eine Gewebeanalyse, bei der das durch die Einwirkung eines elektrischen Funken auf Gewebe erzeugte Licht mittels einer Lichtaufnahmeeinrichtung aufgenommen wird, aus dem aufgenommenen Licht mit einer Spektrometereinrichtung Lichtintensitäten bei verschiedenen Lichtwellenlängen bestimmt werden, und aus den Lichtintensitäten eine Gewebeeigenschaft charakterisierende Daten ermittelt werden. Ausserdem ist eine Reinigungseinrichtung, beispielsweise in Form eines Fluidkanal, vorgesehen. Dieser dient der Zuführung von Fluid zum distalen Ende des Instruments und somit zur Verhinderung von Verschmutzung der Lichteintrittsfenster durch Rauchpartikel, Aerosol und Gewebestücke.

Die Ermittlung der Art des behandelten Gewebes oder sonstiger Gewebeeigenschaften, wie zum Beispiel gutartig oder bösartig, ist auch aus der EP 2 659 846 B1 bekannt. Zur Gewebeerkennung wird dort das Licht eines auf biologisches Gewebe einwirkenden Funkens hinsichtlich seiner spektralen Zusammensetzung untersucht. Auch hier ist die Aufnahme des von dem Funken ausgehenden Lichts in der Nähe des Funkens erforderlich.

Es ist bekannt, dass sich bei Durchführung von Eingriffen am lebenden Gewebe mittels Funkens an Lichteintrittsfenstern Ablagerungen bilden können, die die Gewebeanalyse beeinträchtigen. Zur Abhilfe schlägt die EP 2 815 713 B1 vor, das Lichteintrittsfenster durch einen ruhenden oder strömenden Flüssigkeitskörper zu bilden. Dies soll der Tendenz zur Ablagerung von Ruß oder anderen Verschmutzungen auf dem Lichteintrittsfenster entgegenwirken. Jedoch haben Flüssigkeitskörper keine geometrisch bestimmte Form und sie können insbesondere bei Plasmaanwendungen, die mit hoher Wärmeentwicklung einhergehen, keine Anwendung finden.

Aus diesen und anderen Einflussfaktoren ergibt sich bei der optischen Bestimmung von Gewebeeigenschaften anhand des von einem Funken oder einem Plasma ausgehenden Lichts eine gewisse situationsabhängige Unsicherheit.

Es ist Aufgabe der Erfindung, ein Konzept zur Erfassung der Verlässlichkeit der Gewebeerkennung anzugeben.

Diese Aufgabe wird mit der Einrichtung nach Anspruch 1 und ebenso mit dem Verfahren nach Anspruch 14 gelöst:
Die erfindungsgemäße Gewebeanalyseeinrichtung dient der Gewebeerkennung. Zur Gewebeerkennung wird die spektrale Zusammensetzung von Licht ausgewertet, das durch Einwirkung eines Funkens auf biologisches Gewebe entsteht. Die Gewebeanalyseeinrichtung weist dazu eine Lichtaufnahmeeinrichtung zur Aufnahme von Licht auf, das durch die die Einwirkung eines elektrischen Funkens oder Plasmas auf das biologische Gewebe erzeugt ist. Die Lichtaufnahmeeinrichtung kann zum Beispiel die Endfläche einer optischen Faser, ein dort angebrachtes Objektiv oder dergleichen, sein. Insbesondere ist die Lichtaufnahmeeinrichtung vorzugsweise in der Nähe einer Elektrode eines entsprechenden chirurgischen Instruments angeordnet und somit nahe bei dem entstehenden Funken oder Plasma. Deswegen kann die Lichtaufnahmeeinrichtung einer gewissen Verschmutzung oder auch einer Degradation ausgesetzt sein. Die Verschmutzung kann sich durch Ablagerungen von Ruß, Gewebepartikeln, Staub, Salzkristallen oder ähnlichem ergeben. Das von der Lichtaufnahmeeinrichtung erfasste Licht wird einer Spektrometereinrichtung zugeführt, die die Lichtintensitäten bei mindestens einer, vorzugsweise mehreren, Wellenlängen des Lichts bestimmten und Signale an eine Auswerteeinrichtung ergibt, die die Lichtintensitäten kennzeichnen. Die Auswerteeinrichtung ermittelt aus den die Lichtintensitäten kennzeichnenden Signalen Daten, die wenigstens eine Gewebeeigenschaft kennzeichnen. Unter Gewebeeigenschaft versteht sich jedes ein Gewebe charakterisierende Merkmal, wie beispielsweise Gewebeart (Knochen, Blut, Bindegewebe, Muskel, Nerven, Organgewebe usw. oder auch verschiedene Tumorgewebe). Eine zu unterscheidende Gewebeeigenschaft kann auch eine innerhalb eines Gewebetyps liegenden Eigenschaft sein, zum Beispiel, ob es sich um gesundes oder entzündetes Gewebe, Tumorgewebe, infiziertes Gewebe, abgestorbenes Gewebe oder dergleichen handelt.

Zu der Gewebeanalyseeinrichtung gehört eine Zuordnungseinrichtung zur Zuordnung eines Verlässlichkeitswerts zu den von der Auswerteeinrichtung ermittelten Daten. Die Zuordnungseinrichtung ist dazu eingerichtet, den Verlässlichkeitswert anhand der Verschmutzung der Lichtaufnahmeeinrichtung zu ermitteln. Die Erfassung der Verschmutzung erfolgt mittelbar, indem das von der Spektrometereinrichtung erzeugt Spektrum ausgewertet wird. Das Spektrum kann von einer Lichtquelle bekannter spektraler Zusammensetzung oder auch von dem Licht herrühren, das ein auf Gewebe einwirkender Funke emittiert. Rührt das Licht von einer Lichtquelle bekannter spektraler Zusammensetzung her, ist die Auswertung besonders einfach. Aus dem von der Spektrometereinrichtung gelieferten Spektrum kann dann unmittelbar auf die Art und Stärke der Verschmutzung geschlossen und somit die Verschmutzung klassifiziert werden. Verschiedenen Verschmutzungsklassen können verschiedenen Transmissionskurven entsprechend, die wie Filterkurven das von der Lichtquelle abgegebene und von der Lichtaufnahmeeinrichtung aufgenommenes Licht beeinflussen. Verschiedenen Transmissionskurven können Verlässlichkeitswerte für verschiedene Gewebeeigenschaften beispielsweise verschiedene Gewebetypen zugeordnet sein. Beispielsweise lassen sich bestimmte Gewebetypen auch bei starker Verschmutzung noch relativ sicher erkennen während andere Gewebetypen schon bei geringer Verschmutzung nicht mehr sicher erkannt werden können.

Allerdings muss bei dieser Vorgehensweise zur Verschmutzungserfassung von Zeit zu Zeit eine Testeinrichtung genutzt werden, um die Lichtaufnahmeeinrichtung mit Licht bekannter spektraler Zusammensetzung zu versorgen. Zu der Testeinrichtung kann eine Lichtquelle gehören, die so angeordnet ist, dass von ihr emittiertes Licht von der Lichtaufnahmeeinrichtung erfasst wird. Die Lichtquelle kann beispielsweise eine Lichtquelle sein, die immer dann leuchtet, wenn an der Elektrode eines entsprechenden Instruments kein Funke vorhanden ist. Wenn ein Funke vorhanden ist, kann sie abgeschaltet (nichtleuchtend) sein.

Alternativ kann die Testeinrichtung als Lichtquelle die Operationsfeldbeleuchtung nutzen. Dies gilt insbesondere, wenn die Operationsfeldbeleuchtung Licht in dem für die Gewebeerkennung relevanten Wellenlängenbereich emittiert. Außerdem ist es vorteilhaft, wenn die Helligkeit der Operationsfeldbeleuchtung ausreichend konstant ist. Es ist möglich, den Test dann, gegebenenfalls auch immer dann, auszuführen, wenn das Instrument gerade nicht aktiviert ist.

Bei einer anderen Ausführungsform entfällt eine solche Lichtquelle bzw. wird die Operationsfeldbeleuchtung nicht zu Testzwecken genutzt. Vielmehr wird der Spektrometereinrichtung das von dem Funken emittierte und von der Lichtaufnahmeeinrichtung aufgenommene Licht zugeführt, die das zugehörige Spektrum bestimmt. Die Zuordnungseinrichtung kann dann mit einem Datenblock verbunden sein, der mehrere Transmissionskurvenmodelle enthält, die für verschiedene Verschmutzungen charakteristisch sind. Die Transmissionskurvenmodelle sind wiederum Filterkurven, die sich in ihrer qualitativen Form und in ihren wellenlängenabhängigen Dämpfungswerten unterscheiden können. Die Zuordnungseinrichtung ist in diesem Fall darauf eingerichtet, das zu dem aufgenommenen Spektrum passende Transmissionskurvenmodell zu identifizieren.

Bei beiden Ausführungsformen ist dem Transmissionskurvenmodell ein Datensatz zugeordnet, der verschiedenen Gewebeeigenschaften verschiedene Verlässlichkeitswerte zuordnet. Diese Daten werden zur weiteren Auswertung des Spektrums verwendet. Setzt der Hersteller oder der Anwender beispielsweise einen Verlässlichkeitswert von mindestens 98% fest und weist das im Beispielfall geltende Transmissionskurvenmodell lediglich für einige der prinzipiell bestimmbaren Gewebeeigenschaften eine Verlässlichkeit oberhalb dieser Grenze auf, kann die Zuordnungseinrichtung darauf eingerichtet sein, nur jene Gewebetypen anzuzeigen, für die eine ausreichende Verlässlichkeit gegeben ist. Alternativ können bei jeder erfassten Gewebeeigenschaft die zugehörigen Verlässlichkeitswerte angezeigt werden. Niedrige Verlässlichkeiten können optisch oder akustisch signalisiert werden, um Fehlbehandlungen zu vermeiden.

Weitere Einzelheiten vorteilhafter Ausführungsformen der Erfindung ergeben sich aus Unteransprüchen, aus den Figuren, der Zeichnung oder aus der zugehörigen Beschreibung. Es zeigen:
Figur 1 eine Gewebeanalyseeinrichtung, in schematischer Blockdarstellung,
Figur 2 einen Ausschnitt der Gewebeanalyseeinrichtung zur Verdeutlichung der grundsätzlichen Funktionsweise,
Figur 3 Transmissionskurvenmodelle für verschiedene Verschmutzungen der Lichtaufnahmeeinrichtung,
Figur 4 die räumliche Darstellung eines Verlässlichkeitswerts in Abhängigkeit von Verschmutzungstyp und Ausmaß (Grad) der Verschmutzung,
Figur 5 verschiedene Verlässlichkeitsverläufe bei zunehmender Verschmutzung für verschiedene Gewebeeigenschaften,
Figur 6 eine Gewebeanalyseeinrichtung mit Testeinrichtung als Blockbild.

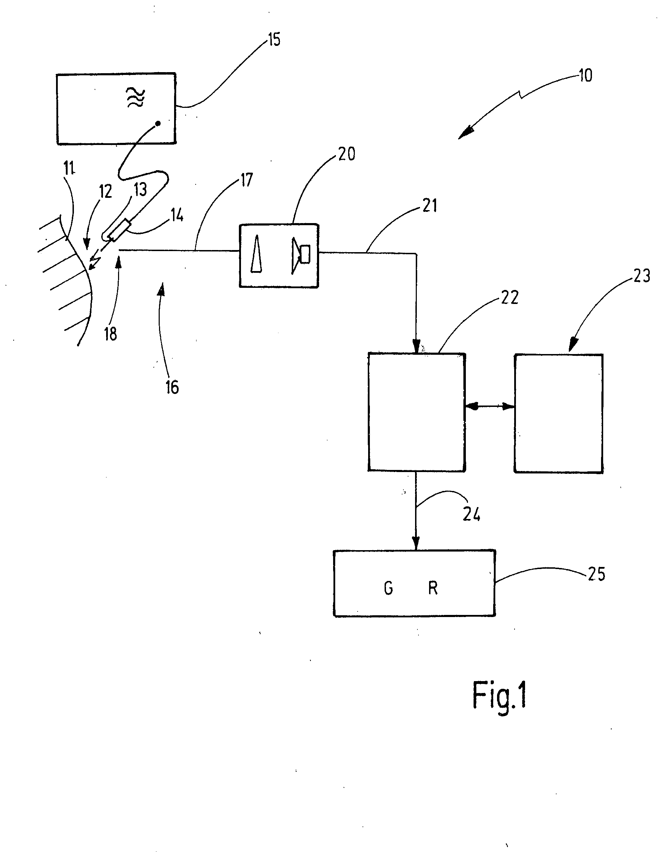

Figur 1 veranschaulicht eine Gewebeanalyseeinrichtung 10, die dazu eingerichtet ist, bestimmte Gewebeeigenschaften G aufgrund von Licht zu erkennen, das von einem auf biologisches Gewebe 11 einwirkenden Funken 12 herrührt. Der Funken 12 kann von einer Elektrode 13 eines chirurgischen Instruments 14 ausgehen, das von einem Gerät 15 mit elektrischer Leistung versorgt wird. Beispielsweise wird die Elektrode 13 dadurch mit hochfrequentem Strom gespeist, der über das Gewebe 11 und eine entsprechende Gegenelektrode abfließt. Es handelt sich bei dem Instrument 14 zum Beispiel um ein monopolares Instrument, das eine am Patienten anzubringende in Figur 1 nicht veranschaulichte Neutralelektrode erfordert, um den Stromkreis zu schließen. Die Gewebeanalyseeinrichtung 10 kann aber auch mit einem bipolaren Instrument zusammenwirken, das zwei oder mehrere Elektroden aufweist, zwischen denen der Funke brennt. Sobald der Funke 12 oder ein von diesem erzeugter Plasmastrahl das Gewebe 11 berührt, entsteht eine Lichterscheinung, deren Spektrum Rückschlüsse auf die Art und den Zustand, d.h., auf Eigenschaften des Gewebes 11 zulässt.

Zur Bestimmung solcher Gewebeeigenschaften G dient die Gewebeanalyseeinrichtung 10, die Teil des Instruments 14 oder auch als gesonderte Einheit aufgebaut sein kann. Die Gewebeanalyseeinrichtung 10 ist darauf eingerichtet, eine fragliche Eigenschaft des Gewebes zu ermitteln und anzuzeigen, z.B. um welche Gewebeart es sich bei dem mit dem Funken in Berührung stehenden Gewebe handelt (z.B. Bindegewebe oder Organgewebe).

Zu der Gewebeanalyseeinrichtung 10 gehört eine Lichtaufnahmeeinrichtung 16, beispielsweise in Gestalt eines Lichtleiters 17, dessen distales Ende 18 ein Lichtaufnahmefenster bildet und in der Nähe der Elektrode 13 und/oder des Funkens 12 angeordnet ist. Das Lichtaufnahmefenster kann auch durch eine Linse, ein Objektiv oder ähnliches gebildet sein.

Die Lichtaufnahmeeinrichtung 16 ist an eine Spektrometereinrichtung 20 angeschlossen und leitet dieser das aufgenommene von dem Funken 12 herrührende Licht zu. Die Spektrometereinrichtung 20 ist dazu eingerichtet, das Spektrum des Lichts zu bestimmen. Das Spektrum ist durch die bei verschiedenen Wellenlängen des Lichts vorhandenen Lichtintensitäten charakterisiert. Als Spektrometereinrichtung 20 eignet sich dabei jede Art eines Spektrometers, die dazu geeignet ist, auf einer Leitung 21 Signale abzugeben, die die verschiedenen Lichtintensitäten bei verschiedenen Lichtwellenlängen kennzeichnen.

Die Leitung 21 verbindet die Spektrometereinrichtung 20 mit einer Auswerteeinrichtung 22, die dazu eingerichtet ist, aus dem von der Spektrometereinrichtung 20 gemessenen Spektren (das heißt, aus den von ihr abgegebenen Signalen) Gewebeeigenschaften G zu bestimmen. Die zu bestimmenden Gewebeeigenschaften G können der Gewebetyp oder auch spezifische Merkmale eines Gewebetyps sein. Beispielsweise kann ein Gewebetyp (Muskelgewebe, Knochengewebe, Fettgewebe, Blut, usw.) auf bestimmte Merkmale hin (Eisengehalt, Phosphorgehalt oder andere subtilere Merkmale) untersucht werden. Die Auswerteeinrichtung kann dazu beispielsweise anhand vieler verschiedener Gewebeproben trainiert werden und entsprechende Lernalgorithmen oder andere lernende Strukturen aufweisen. Die Auswerteeinrichtung 22 kann dazu auch explizit angegebene Rechenalgorithmen oder sonstige Auswertealgorithmen nutzen. Die Auswerteeinrichtung 22 erzeugt Daten D, die Eigenschaften des Gewebes charakterisieren. Die Daten D können beispielsweise dazu geeignet sein, den Gewebetyp zu benennen, malignes von benignem Gewebe zu unterscheiden oder dergleichen.

Die Gewebeanalyseeinrichtung 10 umfasst außerdem eine Zuordnungseinrichtung 23, die dazu eingerichtet ist, den Daten D Verlässlichkeitswerte R zuzuordnen. Sowohl die Daten D als auch die Verlässlichkeitswerte R können über eine Leitung 24 an eine Displayeinrichtung 25 gegeben werden. Der Verlässlichkeitswert wird aufgrund einer Transmissionsmessung bestimmt und gilt für alle kommenden Daten D, bis zur nächsten Transmissionsmessung. Somit werden den Messungen quasi schon im Voraus eine Verlässlichkeit der Gewebeklassifizierung und gegebenenfalls auch eine reduzierte Verlässlichkeit zugeordnet.

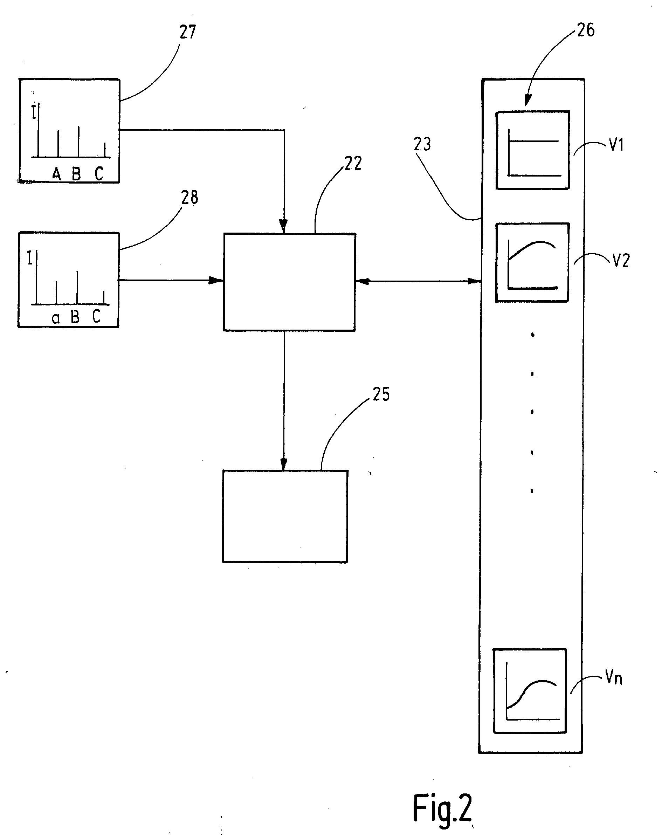

Die Auswerteeinrichtung 22, die mit ihr verbundene Zuordnungseinrichtung 23 und deren Zusammenspiel sind aus Figur 2 näher ersichtlich. Die Zuordnungseinrichtung 23 dient insbesondere dazu, zu ermitteln, wie verlässlich eine bestimmte Gewebeeigenschaft G bestimmbar ist. Tendenziell nimmt die Verlässlichkeit mit zunehmender Verschmutzung der Lichtaufnahmeeinrichtung 17 ab. Jedoch gilt dies nicht für alle zu bestimmenden Gewebeeigenschaften G gleichermaßen. Während zum Beispiel Fettgewebe von Knochengewebe auch dann noch gut unterscheidbar ist, wenn die Lichtaufnahmeeinrichtung bzw. deren Lichteintrittsfenster schon einer eheblichen Verschmutzung unterliegen, kann beispielsweise die subtilere Unterscheidung von ähnlichen Gewebetypen oder die Unterscheidung von benignem und malignem Gewebe bei schon geringeren Verschmutzungsgraden unzuverlässig werden.

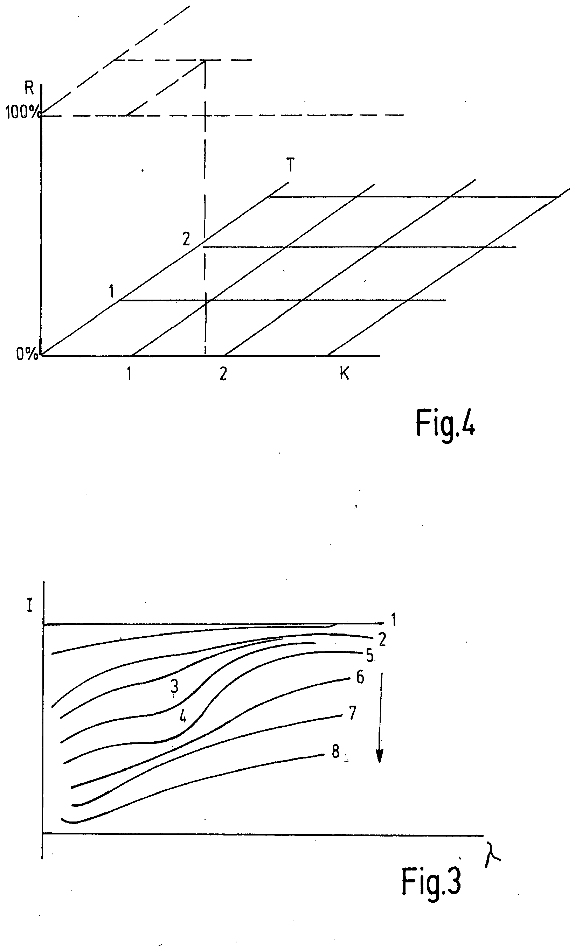

Die Verschmutzung eines Lichteintrittsfensters verändert die Transmissionseigenschaften desselben. Ein Belag auf dem Lichteintrittsfenster wirkt ähnlich wie ein Filter und wirkt somit spektrumsverfälschend. Die Zuordnungseinrichtung kann dazu eine Vielfalt von Transmissionskurvenmodellen 26 bereithalten, die für verschiedene Verschmutzungen V (V1, V2 ... Vn) charakteristisch sind. Während beispielsweise die Transmissionskurve für keine Verschmutzung V1 eine Allpasscharakteristik hat, sind die Transmissionskurven V2 ... Vn Transmissionskurven mit Tiefpass- oder Bandpasscharakteristik oder mit Filterkurven mit mehreren Minima, Maxima und/oder Wendepunkten. Figur 3 veranschaulicht dazu die typische Veränderung der Transmissionskurven bei von 1 bis 9 zunehmender Verschmutzung. Die Kurven zeigen die Lichtintensität I aufgetragen über der Lichtwellenlänge A.

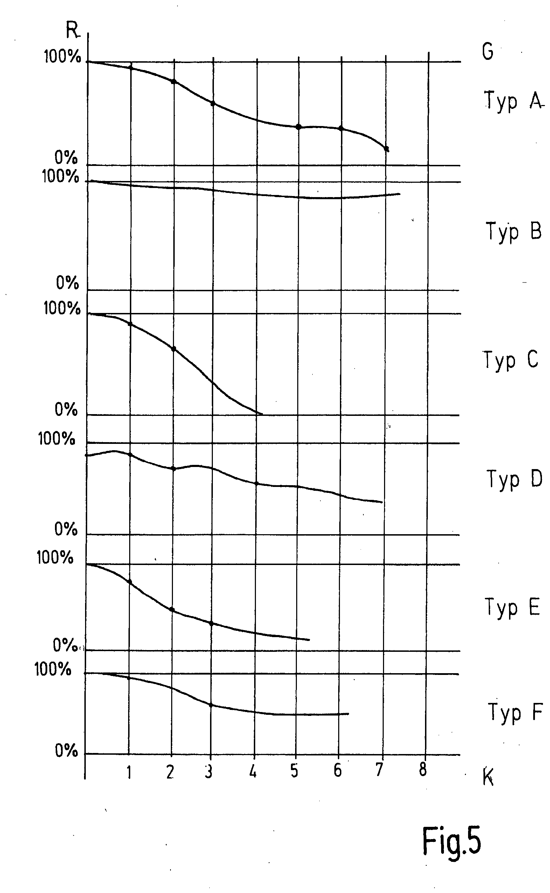

Zu jedem Transmissionskurvenmodell 26 mit den verschiedenen Verschmutzungen V1 bis Vn ergeben sich unterschiedliche Verlässlichkeitswerte R bei der Erkennung von Gewebeeigenschaften G. Dies ist in Figur 5 für verschiedene Gewebetypen Typ A bis Typ F veranschaulicht. Es werden wieder Verschmutzungen, wie in Figur 3 auch hier von 1 bis 8 durchnummeriert, was zunehmenden Verschmutzungsgraden entspricht. Bei einem Verschmutzungsgrad von 1 ist bei fast allen Gewebetypen außer den Typen C und E noch eine Verlässlichkeit von fast 100% gegeben. Mit zunehmenden Verschmutzungsgrad (2, 3, 4, usw.) nimmt die Verlässlichkeit gewebetypabhängig ab, wobei die Abnahmen unterschiedlich stark sind. Dies gilt sowohl für die Typen A bis F der Gewebe wie auch für andere Gewebeeigenschaften G oder Gewebemerkmale. Bei einem gegebenen Verschmutzungsgrad von beispielsweise 3 können beispielsweise einige Gewebeeigenschaften G, zum Beispiel die Eigenschaft Typ B noch sicher, Typ D noch einigermaßen sicher und andere Typen oder Eigenschaften nicht mehr bestimmt werden. Diese Information, nämlich welche Gewebeeigenschaft G mit welcher Sicherheit bestimmbar ist, gehört zu jedem Transmissionskurvenmodell 26 von V1 bis Vn.

Die Auswerteeinrichtung 22 bestimmt zunächst die gewünschte Gewebeeigenschaft G, beispielsweise den Gewebetyp. Die Zuordnungseinrichtung 23 ordnet dieser Eigenschaft dann anhand des jeweils gültigen Transmissionskurvenmodells 26 (V1, V2 ... oder Vn) einen entsprechenden Verlässlichkeitswert R zu. Beide Daten können über die Leitung 24 zu der Displayeinrichtung 25 gegeben und dort angezeigt werden. Die Daten D kennzeichnen dabei zum Beispiel den identifizierten Gewebetyp oder eine sonstige Gewebeeigenschaft G. Der Verlässlichkeitswert R kennzeichnet dabei die Verlässlichkeit mit der die Gewebeeigenschaft G bestimmt worden ist.

Die Bestimmung des Verlässlichkeitswertes R kann, zumindest bei einer Ausführungsform der Erfindung vor der eigentlichen Anwendung stattfinden. Damit können später auch den während der Operation gemessenen Transmissionen Verlässlichkeitswerte R zugeordnet werden. Z.B. können vor der Anwendung an einem Testgewebe mit einer Faser mit 100% Transmission Spektren aufgenommen und das Gewebe klassifiziert werden. Danach können verschiedene Transmissionskurvenmodelle genutzt werden, um verschiedene Verschmutzungen zu simulieren. Mit diesen Transmissionskurvenmmodellen kann dann das Gewebe erneut klassifiziert werden. Durch Vergleich mit dem bei 100% Transmission klassifizierten Gewebe kann festgestellt werden, welches Transmissionskurvenmodell hinsichtlich der Verlässlichkeit der Gewebeanalyse um wieviel schlechter ist.

Das kann ich dann während der Anwendung nutzen, um zu entscheiden, ob eine Faser mit einer bestimmten, während der Anwendung gemessenen Transmission, noch gut genug für die aktuelle Gewebeklassifikation ist.

Es ist auch möglich, die Anzeige von (Daten D, die) Gewebeeigenschaften G zu blockieren, falls der Verlässlichkeitswert R eine festgelegte oder gewählte Grenze unterschreitet.

Die Transmissionskurvenmodelle 26 können eindimensionale Modelle sein, die den in Figuren 3 und 5 nahegelegt lediglich zunehmende Verschmutzung kennzeichnen. Es ist jedoch auch möglich, die Klassifizierung der verschiedenen Verschmutzungen so anzulegen, dass Verschmutzungstypen T und Verschmutzungsausmaß der -grad K unterschieden werden. Beispielsweise kann der Verschmutzungstyp von der Art der Verschmutzung (Kohlenstoffablagerung, Ablagerung von Geweben, Ablagerung von sonstigem Rauch) abhängen. Dies kann insbesondere der Fall sein, wenn die entsprechende Partikelgröße unterschiedlich ist. Das Verschmutzungsausmaß K kann dann die Dicke der gebildeten Ablagerung charakterisieren. Zum Beispiel können die Verschmutzungstypen T verschiedene Filterkurven zum Beispiel Tiefpass oder Bandpass oder eine Kombination aus verschiedenen Grundcharakteristika festlegen, während der Verschmutzungsgrad Eckfrequenzen, Steilheiten oder andere Parameter der Filterkurven charakterisiert.

Die Zuordnungseinrichtung 23 muss aus den vorhandenen Transmissionskurvenmodellen 26 dasjenige Model auswählen, das am besten zu der jeweiligen Verschmutzung passt. Dazu wird auf Figur 2 verwiesen. Mit einem ersten Spektrum 27 soll ein Spektrum symbolisiert werden, wie es bei unverschmutzter Lichtaufnahmeeinrichtung 16 an einem bestimmten Gewebetyp, beispielsweise Muskelgewebe, aufgenommen wird. Es sind mehrere Spektrallinien A, B, C vorhanden, die mit unterschiedlichen Intensitäten I auftreten. Die Anzahl der Spektrallinien und deren Intensitäten hängen vom jeweiligen Gewebetyp ab. In Figur 2 sind diese lediglich symbolisch veranschaulicht. Wird nun wegen einer Verschmutzung der Lichtaufnahmeeinrichtung 16 an dem betroffenen Gewebe, beispielsweise Muskelgewebe, ein anderes Spektrum 28 aufgenommen, sind dessen Spektrallinie aufgrund der Verschmutzung verändert. Während das Spektrum 27 die Spektrallinien A, B, C zeigt weist das Spektrum 28 die Spektrallinien a, B, C auf, d.h., eine oder mehrere Spektrallinien weisen eine geringere Intensität I auf als es bei einer sauberen Lichtaufnahmeeinrichtung 16 der Fall wäre. Ist dem Operateur jedoch bekannt, von welchem Gewebe das Spektrum gewonnen worden ist und ist diese Information für die Auswerteeinrichtung 22 verfügbar, kann diese anhand der Veränderung des Spektrums 28 gegenüber einem idealen Spektrum 27 bestimmen, welches der Transmissionskurvenmodelle 26 die Veränderung des Spektrums bewirkt haben muss und ein entsprechendes Transmissionskurvenmodell aus der Menge der verfügbaren Modelle V1 bis Vn auswählen. Mit der Auswahl des Transmissionskurvenmodells erhält die Auswerteeinrichtung 22 von der Zuordnungseinrichtung 23 zugleich eine Bewertung der Verlässlichkeit, mit der bestimmte Gewebeeigenschaften erkannt werden. Weil zu dem Transmissionskurvenmodell die Verlässlichkeitswerte R für alle anderen Gewebeeigenschaften G bekannt sind, kann der Operateur mit seinem Instrument fortwährend arbeiten und verschiedene Gewebetypen bearbeiten, wobei ihm die Displayeinrichtung 25 immer die erfasste Gewebeeigenschaft G (z.B. den Gewebetyp) und zugehörig den Verlässlichkeitswert R anzeigt, mit der die Gewebeeigenschaft G (z.B. der Gewebetyp) erkannt worden ist.

Die Wahl der entsprechenden Transmissionskurvenmodelle kann nach festgelegten Zeitintervallen, zum Beispiel jeweils nach ein oder mehreren Sekunden überprüft werden. Es ist auch möglich, aufgrund der Aktivierungszeit und der bisherigen Verschmutzungsrate ein Transmissionsmodell zu extrapolieren. Diese Extrapolation kann in festgelegten Zeitintervallen oder bei sich bietenden Gelegenheiten, z.B. zwischen Aktivierungen des Instruments, mit einer Transmissionsmessung überprüft werden. Operateur muss dazu keine gesonderte Kalibrierung vornehmen.

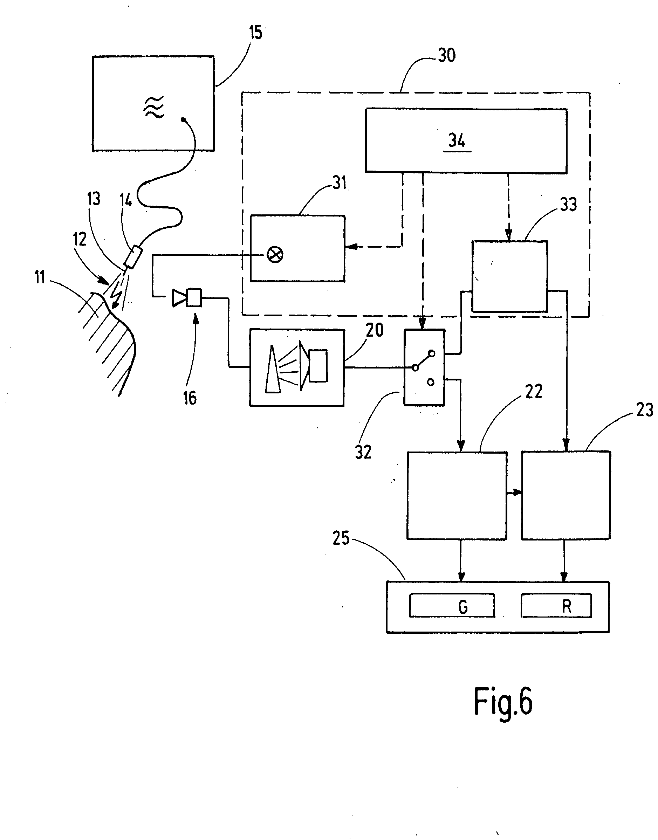

In einer Abwandlung der Erfindung ist es auch möglich, eine Testeinrichtung 30 vorzusehen, wie es Figur 6 veranschaulicht. Diese umfasst eine Lichtquelle 31, mit der dem Lichtaufnahmefenster der Lichtaufnahmeeinrichtung 16 Licht mit definierter spektraler Zusammensetzung zuleitbar ist. Eine Steuereinrichtung 34 koordiniert dabei den Testvorgang, indem sie von Zeit zu Zeit die Lichtquelle 31 aktiviert, sodass das Licht auf das Lichteintrittsfenster der Lichtaufnahmeeinrichtung 16 fällt. Das Spektrometer 20 liefert seine Daten in diesem Testfall über einen Umschalter 32 an eine Transmissionsklassifikator 33, der den Verschmutzungsgrad K und/oder den Verschmutzungstyp T ermittelt. Die Initiierung eines Tests und die Steuerung des Ablaufs desselben obliegt einer Steuereinrichtung 34, die Teil der Testeinrichtung 30 sein kann.

Alternativ kann als Lichtquelle 31 auch die Operationsfeldbeleuchtung genutzt werden. Dazu können zum Beispiel kurze Arbeitspausen genutzt werden, in denen die Elektrode 13 keinen Funken 12 abgibt. Die Steuereinrichtung 34 kann diese Arbeitspausen nutzen und jeweils eine Routine zur Bestimmung des geeigneten Transmissionskurvenmodells abarbeiten.

Der von dem Transmissionsklassifikator 33 gelieferte Verschmutzungsgrad K und/oder Verschmutzungstyp T wird an die Zuordnungseinrichtung 23 geliefert, die wiederum analog zu der vorigen zu Figur 2 gegebenen Beschreibung das passende Transmissionskurvenmodell 26 auswählt. Die Transmissionsmessung liefert Information über den Verschmutzungsgrad. Der Transmissionsklassifikator 33 und/oder die Zuordnungseinrichtung 23 können dazu eingerichtet sein, ein vergleichbares Transmissionskurvenmodell zu bestimmen (aus einem Vorrat auszuwählen) und zu bestimmen, welche Gewebeklassifizierungen mit welcher Verlässlichkeit R damit möglich sind. Dabei können der Transmissionsklassifikator 33 und/oder die Zuordnungseinrichtung 23 darauf eingerichtet sein, anhand des Verschmutzungsgrads und/oder des Verschmutzungstyps eine Interpolation zwischen vergleichbaren Transmissionskurvenmodellen vorzunehmen.

Ist der Test beendet, schaltet die Steuereinrichtung 34 den Umschalter 32 wieder um, sodass die von dem Spektrometer 20 gelieferten Signale an die Auswerteeinrichtung 22 gelangen, die aus den nun wieder aus dem Funkenlicht gewonnenen Spektrum die gewünschten Gewebeeigenschaften ermitteln. Diese Gewebeeigenschaften werden in Form von Daten an die Displayeinrichtung 25 gegeben, die die Gewebeeigenschaften anzeigt.

Somit können der Transmissionsklassifikator und die Zuordnungseinrichtung 23 aus einer gemessenen Transmission eine Vorhersage treffen, wie gut das Ergebnis einer Gewebeklassifizierung sein wird. Z.B. wird mit der aktuell verschmutzten Faser die Gewebeklassifizierung zu 92 % das richtige Ergebnis liefern. Bei sauberer Faser liefert der Gewebeklassifizierer 22 z.B. zu 96 % das richtige Ergebnis. Mittels des Transmissionsklassifikator 33 können nun gemessene Transmissionen unterteilt werden in welche, bei denen die z.B. 92% und besser erreicht werden, und welche, bei denen die zu erwartende Güte der Gewebeklassifizierung unter den 92 % liegt.

Zur Gewebebestimmung werden die Daten D an Gewebeklassifizierer 22 gegeben, der den Gewebetyp bestimmt. Dieser und der ermittelte Verlässlichkeitswert R werden nun an die Displayeinrichtung 25 geliefert. Diese kann den Verlässlichkeitswert R zur Anzeige bringen. Sie kann auch signalisieren, wenn ein Grenzwert unterschritten wird. Der Grenzwert kann fest oder variabel vorgegeben sein.

Eine erfindungsgemäße Gewebeanalyseeinrichtung 10 mit Lichtaufnahmeeinrichtung 16 und Spektrometereinrichtung 20 zur Bestimmung von Gewebeeigenschaften G weist dazu eine Auswerteeinrichtung 22 auf, die mit einer Zuordnungseinrichtung 23 verbunden ist. Die Auswerteeinrichtung 22 dient der Ermittlung wenigstens einer Gewebeeigenschaft G eines biologischen Gewebes, beispielsweise seines Typs oder eine Krankheitsbefalls. Die Zuordnungseinrichtung dient zur Zuordnung eines geeigneten Transmissionskurvenmodells 25, das die Verschmutzung der Lichtaufnahmeeinrichtung 16 modelliert. Für verschiedene Verschmutzungsgrade werden verschiedene Transmissionskurvenmodelle bereitgehalten, die für jede ermittelbare Gewebeeigenschaft G jeweils Verlässlichkeitswerte R beinhalten. Mit dem erfindungsgemäßen Konzept gelingt nicht nur die Gewebeanalyse, sondern darüber hinaus auch die Angabe mit welcher Verlässlichkeit die Analyse durchgeführt worden ist, d.h., wie vertrauenswürdig die Angabe der Gewebeeigenschaft G ist.

### Bezugszeichen:

- 10: Gewebeanalyseeinrichtung
- 11: biologisches Gewebe
- 12: Funken
- 13: Elektrode
- 14: Instrument
- 15: Gerät
- 16: Lichtaufnahmeeinrichtung
- 17: Lichtleiter
- 18: distales Ende des Lichtleiters 17
- 20: Spektrometereinrichtung
- 21: Leitung
- 22: Auswerteeinrichtung
- G: Gewebeeigenschaft
- D: Daten
- 23: Zuordnungseinrichtung
- 24: Leitung
- 25: Displayeinrichtung
- 26: Transmissionskurvenmodelle
- V, V1 ... Vn: Verschmutzungen/Transmissionskurven
- I: Lichtintensität
- A: Lichtwellenlänge
- T: Verschmutzungstypen
- K: Verschmutzungsausmaß
- R: Verlässlichkeitswert
- 27: erstes Spektrum
- 28: anders Spektrum
- 30: Testeinrichtung
- 31: Lichtquelle
- 32: Umschalter
- 33: Transmissionsklassifikator
- 34: Steuereinrichtung
- 35: Faserkoppler

## Patentansprüche

1. Gewebeanalyseeinrichtung (10), insbesondere zur Integration in ein chirurgisches Instrument (14) und oder ein zur Versorgung des Instruments dienendes Gerät (15),
mit einer Lichtaufnahmeeinrichtung (16) zur Aufnahme von durch die Einwirkung eines elektrischen Funkens (12) oder Plasmas auf Gewebe (11) erzeugten Lichts, mit einer Spektrometereinrichtung (20) zur Bestimmung der Lichtintensitäten bei verschiedenen Wellenlängen des Lichts,
mit einer Auswerteeinrichtung (22) zur Ermittlung von wenigstens eine Gewebeeigenschaft (G) charakterisierenden Daten (D) aus den Lichtintensitäten,
mit einer Zuordnungseinrichtung (23) zur Zuordnung und/oder Ermittlung eines Verlässlichkeitswertes (R) zu der oder für die Gewebeeigenschaft (G) anhand einer ermittelten Verschmutzung (V) der Lichtaufnahmeeinrichtung.

2. Gewebeanalyseeinrichtung nach Anspruch 1, **dadurch gekennzeichnet, dass** die Zuordnungseinrichtung (23) dazu eingerichtet ist, anhand aus den von der Spektrometereinrichtung ermittelten Lichtintensitäten eine Klassifizierung der Verschmutzung (V) vorzunehmen.

3. Gewebeanalyseeinrichtung nach einem der vorstehenden Ansprüche, **dadurch gekennzeichnet, dass** eine Testeinrichtung (30) vorgesehen ist, die dazu eingerichtet ist, die Verschmutzung zu klassifizieren.

4. Gewebeanalyseeinrichtung nach einem der vorstehenden Ansprüche, **dadurch gekennzeichnet, dass** die Klassifizierung die Zuordnung der Verschmutzung (V) zu einer von mehreren Verschmutzungstypen (T) umfasst.

5. Gewebeanalyseeinrichtung nach einem der vorstehenden Ansprüche, **dadurch gekennzeichnet, dass** die Testeinrichtung (30) dazu eingerichtet ist, das Ausmaß (K) der Verschutzung (V) zu erfassen.

6. Gewebeanalyseeinrichtung nach den Ansprüchen 4 oder 5, **dadurch gekennzeichnet, dass** die Zuordnungseinrichtung (23) dazu eingerichtet ist, den Verlässlichkeitswert (R) auf Basis der Verschmutzung (V) zu ermitteln, wobei der Verlässlichkeitswert (R) für die Gewebeeigenschaft (G) spezifisch ist, die von der Auswerteeinrichtung (22) zu ermitteln ist.

7. Gewebeanalyseeinrichtung nach einem der vorstehenden Ansprüche, **dadurch gekennzeichnet, dass** die Zuordnungseinrichtung (23) ein Modell über den Zusammenhang zwischen der Verlässlichkeitswert (R), mit der eine bestimmte Gewebeeigenschaft (G) erkannt wird, und der Verschmutzung (V) enthält.

8. Gewebeanalyseeinrichtung nach Anspruch 5, **dadurch gekennzeichnet, dass** das Modell eine Datensammlung ist.

9. Gewebeanalyseeinrichtung nach einem der vorstehenden Ansprüche, **dadurch gekennzeichnet, dass** die Zuordnungseinrichtung (23) mehrere Transmissionskurvenmodelle (26) aufweist, die für verschiedene Verschmutzungen (V) charakteristisch sind.

10. Gewebeanalyseeinrichtung nach Anspruch 9, **dadurch gekennzeichnet, dass** die Zuordnungseinrichtung (23) anhand des von der Spektrometereinrichtung (20) gewonnenen Spektrums (28) das passende Transmissionskurvenmodell (26) auswählt.

11. Gewebeanalyseeinrichtung nach Anspruch 6 oder 10,
**dadurch gekennzeichnet, dass** jedem Transmissionskurvenmodell (26) für jede Gewebeeigenschaft (G) ein Verlässlichkeitswert (R) zugeordnet ist.

12. Gewebeanalyseeinrichtung nach einem der vorstehenden Ansprüche, **dadurch gekennzeichnet, dass** zur Erfassung der Verschmutzung (V) eine Testeinrichtung (30) vorgesehen ist die eine Lichtquelle (31) umfasst, die ein multispektrales Licht aussendet.

13. Gewebeanalyseeinrichtung nach Anspruch 8, **dadurch gekennzeichnet, dass** die Testeinrichtung (31) darauf eingerichtet ist, die Verschmutzung (V) anhand mehrerer Lichtwellenlängen zu ermitteln.

14. Verfahren zur Gewebeanalyse, insbesondere während eines chirurgischen Eingriffs,
bei dem durch die Einwirkung eines elektrischen Funkens (12) oder Plasmas auf Gewebe (11) erzeugtes Licht mittels einer Lichtaufnahmeeinrichtung (16) aufgenommen wird,
aus dem aufgenommenen Licht Lichtintensitäten (I) bei verschiedenen Lichtwellenlängen (λ) bestimmt werden,
aus den Lichtintensitäten (I) wenigstens eine Gewebeeigenschaft (G) charakterisierende Daten (D) ermittelt werden,
eine Verschmutzung (V) der Lichtaufnahmeeinrichtung (16) ermittelt wird,
anhand der ermittelten Verschmutzung (V) den von der Auswerteeinrichtung (22) ermittelten Daten (D) ein Verlässlichkeitswert (R) für die ermittelte Gewebeeigenschaft (G) zugeordnet wird,
die Gewebeeigenschaft (G) und der zugehörige Verlässlichkeitswert (E) angezeigt werden,
oder
die Gewebeeigenschaft (G) nur dann angezeigt wird, wenn der zugehörige Verlässlichkeitswert (R) einen Schwellwert übersteigt.

15. Verfahren nach Anspruch 14, **dadurch gekennzeichnet, dass** Testintervalle in Abhängigkeit von der zu ermittelnden Gewebeeigenschaft (G) festgelegt werden.

## Claims

1. Tissue analysis device (10), particularly for integration in a surgical instrument (14) and/or an apparatus (15) serving for supply of the instrument,
having a light receiving device (16) for receiving light created by the influence of an electrical spark (12) or plasma on tissue (11),
having a spectrometer device (20) for determination of light intensities at different wavelengths of the light,
having an evaluation device (22) for determination of data (D) from the light intensities characterizing at least one tissue characteristic (G),
having an assignment device (23) for assignment and/or determination of a reliability value (R) referring to or for the tissue characteristic (G) based on a determined contamination (V) of the light receiving device.

2. Tissue analysis device according to claim 1, **characterized in that** the assignment device (23) is configured to carry out a classification of the contamination (V) based on the light intensities determined by the spectrometer device.

3. Tissue analysis device according to any of the preceding claims, **characterized in that** a test device (30) is provided that is configured to classify the contamination.

4. Tissue analysis device according to any of the preceding claims, **characterized in that** the classification comprises the assignment of the contamination (V) to one or more contamination types (T).

5. Tissue analysis device according to any of the preceding claims, **characterized in that** the test device (30) is configured to detect the degree (K) of the contamination (V) .

6. Tissue analysis device according to claims 4 or 5, **characterized in that** the assignment device (23) is configured to determine the reliability value (R) based on the contamination (V), wherein the reliability value (R) is specific for the tissue characteristic (G) that is to be determined by the evaluation device (22).

7. Tissue analysis device according to any of the preceding claims, **characterized in that** the assignment device (23) comprises a model about the relation between the reliability value (R) with which a specific tissue characteristic (G) is recognized and the contamination (V) .

8. Tissue analysis device according to claim 5, **characterized in that** the model is a data collection.

9. Tissue analysis device according to any of the preceding claims, **characterized in that** the assignment device (23) comprises multiple transmission curve models (26) that are characteristic for different contaminations (V).

10. Tissue analysis device according to claim 9, **characterized in that** the assignment device (23) selects the matching transmission curve model (26) based on the spectrum (28) obtained by the spectrometer device (20).

11. Tissue analysis device according to claim 6 or 10, **characterized in that** a reliability value (R) is assigned to each transmission curve model (26) for each tissue characteristic (G).

12. Tissue analysis device according to any of the preceding claims, **characterized in that** a test device (30) is provided for determination of the contamination (V) that comprises a light source (31) that emits multi-spectral light.

13. Tissue analysis device according to claim 8, **characterized in that** the test device (30) is configured to determine the contamination (V) based on multiple light wavelengths.

14. Method for tissue analysis, particularly during a surgical intervention,
during which light is received by means of a light receiving device (16), wherein the light is created due to the influence of an electrical spark (12) or plasma on tissue (11),
wherein light intensities (I) at different light wavelengths (X) are determined from the received light, wherein data (D) are determined characterizing at least one tissue characteristic (G) from the light intensities (I),
wherein a contamination (V) of the light receiving device (16) is determined,
wherein a reliability value (R) for the determined tissue characteristic (G) is assigned to the data (D) determined by the evaluation device (22) based on the determined contamination (V),
wherein the tissue characteristic (G) and the assigned reliability value (R) are indicated or the tissue characteristic (G) is only indicated, if the assigned reliability value (R) exceeds a threshold.

15. Method according to claim 14, **characterized in that** test intervals are defined depending on the tissue characteristic (G) to be determined.

## Revendications

1. Dispositif d'analyse tissulaire (10), destiné en particulier à l'intégration dans un instrument chirurgical (14) et ou un appareil (15) servant à l'alimentation de l'instrument,
comprenant un dispositif de réception de lumière (16) destiné à recevoir de la lumière produite par l'action d'une étincelle électrique (12) ou d'un plasma électrique sur un tissu (11),
comprenant un dispositif de spectromètre (20) destiné à déterminer les intensités lumineuses pour des longueurs d'onde différentes de la lumière,
comprenant un dispositif d'évaluation (22) destiné à déterminer des données (D) caractérisant au moins une propriété tissulaire (G), à partir des intensités lumineuses,
comprenant un dispositif d'attribution (23) destiné à attribuer et/ou déterminer une valeur de fiabilité (R) à la propriété tissulaire (G) ou pour la propriété tissulaire, à l'aide d'une salissure (V) déterminée du dispositif de réception de lumière.

2. Dispositif d'analyse tissulaire selon la revendication 1, **caractérisé en ce que** le dispositif d'attribution (23) est conçu pour procéder à une classification de la salissure (V) à l'aide des intensités lumineuses déterminées par le dispositif de spectromètre.

3. Dispositif d'analyse tissulaire selon l'une des revendications précédentes, **caractérisé en ce qu'**il est prévu un dispositif de test (30) qui est conçu pour classifier la salissure.

4. Dispositif d'analyse tissulaire selon l'une des revendications précédentes, **caractérisé en ce que** la classification comprend l'attribution de la salissure (V) à un ou plusieurs types de salissure (T).

5. Dispositif d'analyse tissulaire selon l'une des revendications précédentes, **caractérisé en ce que** le dispositif de test (30) est conçu pour détecter le degré (K) de la salissure (V).

6. Dispositif d'analyse tissulaire selon les revendications 4 ou 5, **caractérisé en ce que** le dispositif d'attribution (23) est conçu pour déterminer la valeur de fiabilité (R) sur la base de la salissure (V), la valeur de fiabilité (R) étant spécifique de la propriété tissulaire (G) qui doit être déterminée par le dispositif d'évaluation (22).

7. Dispositif d'analyse tissulaire selon l'une des revendications précédentes, **caractérisé en ce que** le dispositif d'attribution (23) contient un modèle concernant la relation entre la valeur de fiabilité (R), avec laquelle est identifiée une propriété tissulaire (G) précise, et la salissure (V).

8. Dispositif d'analyse tissulaire selon la revendication 5, **caractérisé en ce que** le modèle est un recueil de données.

9. Dispositif d'analyse tissulaire selon l'une des revendications précédentes, **caractérisé en ce que** le dispositif d'attribution (23) présente plusieurs modèles de courbes de transmission (26) qui sont caractéristiques de différentes salissures (V).

10. Dispositif d'analyse tissulaire selon la revendication 9, **caractérisé en ce que** le dispositif d'attribution (23) sélectionne le modèle de courbe de transmission (26) pertinent à l'aide du spectre (28) obtenu par le dispositif de spectromètre (20).

11. Dispositif d'analyse tissulaire selon la revendication 6 ou 10, **caractérisé en ce qu'**une valeur de fiabilité (R) est attribuée à chaque modèle de courbe de transmission (26) pour chaque propriété tissulaire (G).

12. Dispositif d'analyse tissulaire selon l'une des revendications précédentes, **caractérisé en ce que** pour la détection de la salissure (V), il est prévu un dispositif de test (30) qui comprend une source lumineuse (31) émettant une lumière multispectrale.

13. Dispositif d'analyse tissulaire selon la revendication 8, **caractérisé en ce que** le dispositif de test (31) est conçu pour déterminer la salissure (V) à l'aide de plusieurs longueurs d'ondes lumineuses.

14. Procédé d'analyse tissulaire, en particulier au cours d'une intervention chirurgicale,
selon lequel de la lumière produite par l'action d'une étincelle électrique (12) ou d'un plasma électrique sur un tissu (11) est recueillie au moyen d'un dispositif de réception de lumière (16),
des intensités lumineuses (I) pour différentes longueurs d'ondes lumineuses (λ) sont déterminées à partir de la lumière recueillie,
des données (D) caractérisant au moins une propriété tissulaire (G) sont déterminées à partir des intensités lumineuses (I),
une salissure (V) du dispositif de réception de lumière (16) est déterminée,
une valeur de fiabilité (R) pour la propriété tissulaire (G) déterminée est attribuée aux données (D) établies par le dispositif d'évaluation (22), à l'aide de la salissure (V) déterminée,
la propriété tissulaire (G) et la valeur de fiabilité (E) associée sont visualisées,
ou
la propriété tissulaire (G) est visualisée uniquement si la valeur de fiabilité (R) associée dépasse une valeur seuil.

15. Procédé selon la revendication 14, **caractérisé en ce que** des intervalles de test sont définis en fonction de la propriété tissulaire (G) devant être déterminée.
